# EUROPEAN PATENT SPECIFICATION

(11) **EP 3 776 631 B1**
(45) Date of publication and mention of the grant of the patent: **09.09.2026**
(21) Application number: 19781823.0
(22) Date of filing: 04.04.2019
(51) Int. Cl.: C23C 18/14

(54) **SPIN-ON METALLIZATION**
AUFSCHLEUDERMETALLISIERUNG
MÉTALLISATION PAR ROTATION

(30) Priority: 06.04.2018 US 201862653753 P; 26.03.2019 US 201916365109
(43) Date of publication of application: 17.02.2021
(73) Proprietor: Versum Materials US, LLC, Tempe, AZ 85284 (US)
(72) Inventor: COOPER, Alan, C., Tempe, AZ 85284 (US); IVANOV, Sergei, V., Tempe, AZ 85284 (US); LI, Hongbo, Tempe, AZ 85284 (US); PEARLSTEIN, Ronald, Martin, Tempe, AZ 85284 (US); LEI, Xinjian, Tempe, AZ 85284 (US)
(74) Representative: Merck Patent Association
(86) International application number: PCT/US2019/025852
(87) International publication number: WO 2019/195590

(56) References cited:
- EP-A1- 3 093 367
- WO-A1-00/12777
- WO-A1-2013/148490
- KR-A- 20070 035 704
- KR-A- 20070 075 172
- US-A- 2 523 461
- US-A- 2 918 392
- US-A1- 2005 064 706
- US-A1- 2014 256 081
- US-A1- 2015 050 431
- US-A1- 2015 255 333
- LEE KEUNWOO ET AL: "Characteristics of Cobalt Films Deposited by Metal Organic Chemical Vapor Deposition Method Using Dicobalt Hexacarbonyl tert -Butylacetylene", JAPANESE JOURNAL OF APPLIED PHYSICS, vol. 47, no. 7, 11 July 2008 (2008-07-11), JP, pages 5396 - 5399, XP055873518, ISSN: 0021-4922, DOI: 10.1143/JJAP.47.5396

## Description

### CROSS REFERENCE TO RELATED APPLICATIONS

This application claims priority to U.S. provisional application 62/653,753 filed on April 6, 2018, the entire contents of which is incorporated herein by reference thereto for all allowable purposes.

### BACKGROUND OF THE INVENTION

The present invention relates to fabrication processing techniques of semiconductor devices and related devices. In particular, it relates to techniques for performing film deposition using a metallic element containing compound as a liquid or as a solution in a suitable solvent.

There are a number of conventional ways to lay down conductive lines or vias in semiconductor devices. One way is to do physical vapor deposition involving physical processes such as evaporation or sputtering of a metal or alloy from a metallic target onto the surface of the semiconductor wafer through the application of heat, ion beam or other energy source. Chemical vapor deposition, wherein a metallic or metal halide precursor in the vapor phase is selectively decomposed or chemically reduced on the surface. A subset of chemical vapor deposition is atomic layer deposition where the metal precursor and reducing agent are sequentially exposed to the surface to grow the metallic film in a layer by layer manner. Other techniques commonly employed include electroplating, wherein the wafer is coated with an electrolyte and connected to a DC electric circuit with the substrate serving as the cathode. When current is passed, metal ions dissolved in the electrolyte are chemically reduced on the surface of the cathode. Other techniques known in the art include electroless deposition (autocatalytic deposition) wherein a mixture of metallic ions and chemical reducing agents dissolved in a solvent are contacted to the substrate. A chemical reaction catalyzed by the surface leads to the reaction of the reducing agent with the metal ions to form a reduced metallic coating.

Examples of interconnect metallization of the prior art: US6048445; US5151168, US5674787.

There are many challenges of the prior art. In particular, many of these techniques, and in particular physical vapor deposition, have significant challenges in completely filling high aspect ratio features (i.e. features that are much deeper than they are wide at the opening). The gas-phase processes are typically also incapable of completely filling re-entrant features (i.e. features that have a narrow opening but expand laterally below the surface). Incomplete filling can lead to spots of high resistivity and cause current fluctuations and also lead to localized heating or exacerbate electromigration.

Atomic Layer Deposition (ALD) can, in principle, fill complex high aspect ratio features, but in practice often leaves a seam where the deposit growing inwards from each side-wall merges. Such seams can likewise lead to undesired defects in the electrical performance of the interconnect circuits.

Electroplating requires that a seed layer be deposited, and as dimensions of the features get smaller as the technology progresses, this becomes increasingly difficult.

Another challenge of the prior art is achieving acceptable electrical conductivity of the interconnect circuit.

US8232647 describes one approach to dealing with so-called keyhole defect formation or seams in conventional metallization.

JP2012012647A2 (WO201163235) by Tokyo Electron discloses use of a spin track under inert atmosphere wherein a solvent borne metal complex is deposited on the surface. This patent focuses on aluminum containing precursor but also discloses that silver, gold or copper. There is no description of preferred on suitable complexes for this application nor the use of zerovalent metal complexes, their pre-agglomeration, preference for using liquid or low melting point complexes. The aluminum compounds referenced were AI(III) hydrides and amine adducts thereof. Such compounds decompose by reductive elimination, i.e. the ligands themselves act as the reducing agent.

US6852626B1 by Applied Materials, also referenced in the above, discloses decomposition of a metallic complex, specifically Cu(I)hfac(tmvs), on the surface to deposit a metallic copper film. Copper metal is formed by disproportionation into Cu(ll) and Cu(O).

US9653306B2 by JSR details the use of a zerovalent Co precursor along with a silicon precursor (a silane or halosilane) to form a self-aligned cobalt silicide thin film.

Maria Careri et al studied high-performance liquid chromatography of trinuclear ruthenium acetylido-carbonyl compounds in Journal of Chromatography, 634 (1993) 143-148.

KR 10-2007-0035704 A discloses a method to deposit a conductive metallic film by applying a solution of Ni(CO)₄ in a solvent to the surface of a substrate and relates to spray deposition of the material on a planar substrate.

US 2005/0064706 A1 discloses a method of forming a cobalt layer on a silicon substrate using a vaporised Co precursor of the formula Co₂(CO)₆(R¹-C≡C-R²).

US 2,523,461 A discloses a coating process using a solution of a metal carbonyl in a hydrocarbon to deposit a metal coating, in particular using nickel carbonyl.

US 2,918,392 A discloses a process of depositing metal in the pores of a porous body using a liquid metal carbonyl.

EP 3093367 A1 discloses a system for storage of a chemical precursor and delivery to a process tool wherein the precursor can be selected from various metal complexes, such as metal carbonyls.

WO 00/12777 A1 discloses a method of manufacturing a semiconductor structure using (diene)Ru(CO)₃ as the liquid precursor.

K. Lee et al. (Japanese J. App. Phys. 2008, 47(7),5396-5399) disclose the chemical vapor deposition of cobalt films using dicobalt hexacarbonyl tert-butylacetylene.

WO 2013/148490 A1 discloses a method for depositing a metal layer in a semiconductor device using a cobalt containing precursor, such as dicobalt hexacarbonyl butylacetylene.

Thus, the development of precursors is necessary and is needed for a high purity film with controlled grain boundaries which maximally fills the circuit paths.

### SUMMARY

Described herein are the depositions of conductive metallic films on a surface which contains topography comprising patterned recesses. The present invention uses a neutral (uncharged) metal compound as the precursor in which the metal atom is in the zerovalent state and stabilized by ligands which are stable as uncharged, volatile species, as defined below.

The invention is directed to a method to deposit a conductive metallic film onto a substrate comprising:
A. providing the substrate with a surface containing topography comprising patterned recesses;
B. providing liquid metallic precursor comprising a neutral uncharged metal compound selected from the group consisting of
   a. R¹Co₂(CO)₆, wherein R¹ is a linear or branched C₂ to C₁₀ alkyne, a linear or branched C₁ to C₁₀ alkoxy alkyne, a linear or branched C₁ to C₁₀ organoamino alkyne, preferably (tert-butylacetylene)dicobalt hexacarbonyl, [C₂(CO)₆HC≡CC(CH₃)₃];
   b. R¹CoFe(CO)₇, wherein R¹ is a linear or branched C₂ to C₁₀ alkyne, a linear or branched C₁ to C₁₀ alkoxy alkyne, a linear or branched C₁ to C₁₀ organoamino alkyne;
   c. R²CCo₃(CO)₉, wherein R² is selected from the group consisting of hydrogen, a linear or branched C₁ to C₁₀ alkyl, a linear or branched C₁ to C₁₀ alkoxy, CI, Br, COOH, COOMe, COOEt;
   d. R²CCo₂Mn(CO)₁₀, wherein R² is selected from the group consisting of hydrogen, a linear or branched C₁ to C₁₀ alkyl, a linear or branched C₁ to C₁₀ alkoxy, CI, Br, COOH, COOMe, COOEt;
   e. R³Co₄(CO)₁₂,wherein R³ is selected from a linear or branched C₁ to C₁₀ alkenylidene or wherein the compound is (1-decyne) tetracobalt dodecacarbonyl (Co₄(CO)₁₂(C₈H₁₇C≡CH)), (1,6-heptadiyne) tetracobalt dodecacarbonyl, or (2,2,6-trimethyl-3-heptyne) dicobalt hexacarbonyl; and
   f. R⁴Ru₃(CO)₁₁, wherein R⁴ is selected from the group consisting of a disubstituted alkyne (R^{#}CCR^{##}) wherein R^{#} and R^{##} can be selected independently from the group consisting of C₁ to C₁₂ linear, branched, cyclic or aromatic halocarbyl or hydrocarbyl radical, silyl or organosilyl radical, stannyl or organostannyl radical, and combinations thereof; and

   the neutral uncharged metal compound is present as a liquid or a solution of a solid soluble at ambient temperature in a solvent selected from the group consisting of saturated linear, branched and cyclic hydrocarbons, o-dichlorobenzene, nitrobenzene; nitriles selected from a group comprising of acetonitrile, propionitrile or benzonitrile; ethers selected from a group comprising of tetrahydrofuran, dimethoxyethane, diglyme, tetrahydropyran, methyltetrahydrofuran, butyltetrahydrofuran, p-dioxane; amines selected from a group comprising of triethylamine, piperidine, pyridine, pyrrolidine, morpholine; amides selected from a group comprising of N,N-dimethylacetamide, N,N-dimethylformamide, N-methylpyrrolidinone, N-cyclohexylpyrrolidinone; aminoethers having formulae R⁴R⁵NR⁶OR⁷NR⁸R⁹, R⁴OR⁶NR⁸R⁹, O(CH₂CH₂)₂NR⁴, R⁴R⁵NR⁶N(CH₂CH₂)₂O, R⁴R⁵NR⁶OR⁷N(CH₂CH₂)₂O, O(CH₂CH₂)₂NR⁴OR⁶N(CH₂CH₂)₂O; wherein R⁴⁻⁹ are independently selected from the group consisting of a linear or branched C₁ to C₁₀ alkyl; and combinations thereof;
      and
   the liquid metallic precursor has a viscosity at ambient temperature between 0.5 cP and 20 cP;
C. applying the liquid metallic precursor to the surface to deposit the conductive metallic film onto the substrate by spray coating, roll coating, doctor blade drawdown by squeegee, spin coating, pooling on the surface, inkjet printing, curtain coating, dip-coating, or combinations thereof; and
D. applying vacuum or an energy to the liquid metallic precursor to dissociate the ligands stabilizing the metal; wherein the energy is selected from the group consisting of visible, infrared or ultraviolet light; a heated gas stream; conduction from a resistively or fluid-heated susceptor; an induction-heated susceptor; electron beams; ion beams; remote hydrogen plasma; direct argon; helium or hydrogen plasma; ultrasound; and combinations thereof.

In order to create conductive paths on a surface which has been patterned with recesses in a semiconductor substrate, a liquid metallic precursor containing a metallic compound, as defined above, as a liquid or as a solution in a suitable solvent is applied to the surface. The pool of liquid may be spread on the surface under inert conditions in a known manner so that the recessed areas are filled with this liquid by capillary action, optionally with excess liquid retained on top of the surface by the surface tension of the liquid. The substrate is then subjected to heating that leads to evaporation of the optional solvent and some of the stabilizing ligands, leading to partial decomposition of the precursor to form agglomerated metallic clusters or nanoparticles that on further heating coalesce in the recesses while they release the bulk of the stabilizing ligands to leave a conductive metallic solid. In a preferred embodiment of this invention, the metallic solid partially or substantially fills the gaps or recesses in high-aspect-ratio or reentrant features initially present on the surface of the substrate, and thereby enabling gap-filling.

The metallic precursors for this process comprise a neutral (uncharged) metal compound having a metal in zerovalent state and at least one neutral stabilizing ligand which can be released as neutral molecules.

The neutral (uncharged) metal compound can be a liquid or a solid which is soluble at ambient temperature (defined as 15 °C to 25 °C), in a solvent selected from the group consisting of saturated linear, branched and cyclic hydrocarbons.

The liquid metallic precursor has a viscosity at ambient temperature between 0.5 cP and 20 cP, preferably between 1 cP and 10 cP, and more preferably between 2 cP and 5 cP.

The liquid metallic precursor is applied to the surface with a contact angle between the metallic precursor and the surface at <90°, preferably <45°, or more preferably <30°.

The method further comprises applying vacuum or an energy to the metallic precursor to dissociate the ligands stabilizing the metal; and the energy is selected from the group consisting of visible, infrared or ultraviolet light; a heated gas stream; conduction from a resistively or fluid-heated susceptor; an induction-heated susceptor; electron beams; ion beams; remote hydrogen plasma; direct argon; helium or hydrogen plasma; ultrasound; and combinations thereof.

The method can additionally comprises applying a post-deposition annealing treatment.

In another aspect, described herein is a system to deposit a conductive metallic film onto a substrate comprising:
a. the substrate with a surface containing topography comprising patterned recesses;
b. the metallic precursor as disclosed above; and
c. a deposition tool selected from the group consisting of spray coating, roll coating, doctor blade drawdown (squeegee), spin coating, pooling on the surface, inkjet printing, curtain coating, dip-coating, and the combinations thereof.

In yet another aspect, described herein is a vessel containing the metallic precursor as disclosed above. The vessel can have a dip-tube extending beneath the surface of the liquid metallic precursor to facilitate the delivering of the precursor to the deposition site.

In yet another aspect, described herein is a conductive metallic film deposited on a surface containing topography comprising patterned recesses by using liquid metallic precursor comprising a neutral uncharged metal compound selected from the group consisting of dicobalthexacarbonyltert-butylacetylene [Co₂(CO)₆HC≡CC(CH₃)₃], (1-decyne) tetracobalt dodecacarbonyl (Co₄(CO)₁₂(C₈H₁₇C≡CH)), (1,6-heptadiyne) tetracobalt dodecacarbonyl, (2,2,6-trimethyl-3-heptyne) dicobalt hexacarbonyl, and (2,2-dimethyl-3-octyne) dicobalt hexacarbonyl (CCTNBA); and a solvent selected from the group consisting of tetrahydrofuran, octane, hexane, toluene, wherein the film is optionally deposited by spray coating, roll coating, spin coating, inkjet printing, dip-coating, and combinations thereof, and wherein the film optionally has an electrical conductivity less than or equal to 1 x 10⁻⁴ Ωcm at ambient temperature.

### BRIEF DESCRIPTION OF THE DRAWINGS

The present invention will hereinafter be described in conjunction with the appended figures wherein like numerals denote like elements:
Figure 1 shows thermogravimetric analysis (TGA) data for (1-decyne)tetracobalt dodecacarbonyl measured under flowing nitrogen.
Figure 2 shows a typical conductive cobalt-containing film deposited on a wafer coupon in current application.

### DETAILED DESCRIPTION

The ensuing detailed description provides preferred exemplary embodiments only, and is not intended to limit the scope, applicability, or configuration of the invention. Rather, the ensuing detailed description of the preferred exemplary embodiments will provide those skilled in the art with an enabling description for implementing the preferred exemplary embodiments of the invention. Various changes may be made in the function and arrangement of elements without departing from the spirit and scope of the invention, as set forth in the appended claims.

In the claims, letters may be used to identify claimed method steps (e.g. a, b, and c). These letters are used to aid in referring to the method steps and are not intended to indicate the order in which claimed steps are performed, unless and only to the extent that such order is specifically recited in the claims.

In order to create conductive paths on a surface which has been patterned with recesses in a dielectric material, a liquid metallic precursor containing a metallic compound, as defined above, as a liquid or as a solution in a suitable solvent, as defined above, is applied to the surface. The pool of liquid may be spread on the surface under inert conditions in a known manner so that the recessed areas are filled with this liquid by capillary action, optionally with excess liquid retained on top of the surface by the surface tension of the liquid. The substrate is then subjected to heating that leads to evaporation of the optional solvent and some of the stabilizing ligands, leading to partial decomposition of the precursor to form agglomerated metallic clusters or nanoparticles that on further heating coalesce in the recesses while they release the bulk of the stabilizing ligands to leave a conductive metallic solid.

This method is particularly advantageous when said topography has a high aspect ratio (aspect ratio may be defined as the depth of the feature (L) multiplied by the perimeter (p) of said feature divided by four times the cross sectional area (A) of the feature where all measurements are in comparable units; AR = [Lp]/[4A]).

The neutral (uncharged) metal compound can most advantageously be a liquid or a solid which has high solubility in a solvent as defined above.

In order to facilitate transport of the metallic precursor into the topography on the surface, it is should be in the form of a low viscosity liquid.

If the neutral (uncharged) metal compound is a solid or viscous liquid at ambient temperature, it is conveniently supplied as a solution in a solvent as defined above. The viscosity of this liquid at ambient temperature is between 0.5 cP and 20 cP, preferably between 1 cP and 10 cP and most preferably between 2 cP and 5 cP.

Examples of terminal or internal alkynes include but are not limited to propyne, 1-butyne, 3-methyl-1-butyne, 3,3-dimethyl-1-butyne, 1-pentyne, 1-hexyne, 1-decyne, cyclohexylacetylene, phenylacetylene, 2-butyne, 3-hexyne, 4,4-dimethyl-2- pentyne, 5,5-dimethyl-3-hexyne, 2,2,5,5-tetramethyl-3-hexyne, trimethysilylacetylene, phenyacetylene, diphenyl acetylene, trichlorosilylacetylene, trifluoromethylacetylene, cyclohexylacetylene, trimethylstannylacetylene.

Examples of neutral (uncharged) metal precursors include more specifically but are not limited to dicobalthexacarbonyl-tert-butylacetylene [Co₂(CO)₆HC≡CC(CH₃)₃], (1-decyne) tetracobalt dodecacarbonyl (Co₄(CO)₁₂(C₈H₁₇C≡CH)), (1,6-heptadiyne) tetracobalt dodecacarbonyl, (2,2,6-trimethyl-3-heptyne) dicobalt hexacarbonyl, (2,2-dimethyl-3-octyne) dicobalt hexacarbonyl (CCTNBA), (2,2-dimethyl-3-decyne) dicobalt hexacarbonyl, (2,2-dimethyl-3-heptyne) dicobalt hexacarbonyl, and (tert-butylmethylacetylene)dicobalt hexacarbonyl (CCTMA).

Some of the precursor as described above may be dissolved in a suitable solvent to render it into a low viscosity liquid.

Suitable solvents are selected from the group of solvents defined above.

Suitable solvents include but are not limited to n-hexane, n-pentane, isomeric hexanes, octane, isooctane, decane, dodecane, heptane, cyclohexane, methylcyclohexane, ethylcyclohexane, decalin; aromatic solvents such as benzene, toluene, xylene (single isomer or mixture of isomers), mesitylene, o-dichlorobenzene, nitrobenzene; nitriles such as acetonitrile, propionitrile or benzonitrile; ethers such as tetrahydrofuran, dimethoxyethane, diglyme, tetrahydropyran, methyltetrahydrofuran, butyltetrahydrofuran, p-dioxane; amines such as triethylamine, piperidine, pyridine, pyrrolidine, morpholine; amides such as N,N-dimethylacetamide, N,N-dimethylformamide, N-methylpyrrolidinone, N-cyclohexylpyrrolidinone; aminoethers having formaulae R⁴R⁵NR⁶OR⁷NR⁸R⁹, R⁴OR⁶NR⁸R⁹, O(CH₂CH₂)₂NR⁴, R⁴R⁵NR⁶N(CH₂CH₂)₂O, R⁴R⁵NR⁶OR⁷N(CH₂CH₂)₂O, O(CH₂CH₂)₂NR⁴OR⁶N(CH₂CH₂)₂O wherein R⁴⁻⁹ are independently selected from the group consisting of a linear or branched C₁ to C₁₀ alkyl and mixtures thereof.

The neat precursor liquid or a solution of precursor in solvent may be applied to a substrate having topographic features comprising patterned recesses by means known in the art, selected from spray coating, roll coating, doctor blade drawdown (squeegee), spin coating, pooling on the surface, inkjet printing, curtain coating and dip-coating.

In order to achieve high quality films, the liquid may be applied to the substrate under a controlled atmosphere which has reduced oxygen or moisture content compared to ambient air. To enable such a process, the metal element containing liquids of the present invention can be contained in a sealed vessel or container, such as the one disclosed in US2002/0108670A1.

The vessel may be connected to deposition equipment known in the art by use of a valved closure and a sealable outlet connection. For convenience, the outlet connection may be connected to a dip-tube extending beneath the surface of the liquid so that the liquid may be delivered to the substrate by the use of a pressure difference.

Most preferably, the vessels may be constructed of high purity materials, including stainless steel, glass, fused quartz, polytetraflurorethylene, PFA^{®}, FEP^{®}, Tefzel^{®} and the like. The vessels may be sealed with one or more valves. The headspace of the vessel is preferably filled with a suitable gas such as nitrogen, argon, helium or carbon monoxide. One or more of the valves may be connected to a dip tube which extends below the surface of the liquid, and one or more of the valves may be in fluid communication with the head space gas.

The liquid applied to the surface will be drawn into the fine topography on the surface due to capillary action. In order to fill fine topographic features, therefore, a contact angle between this liquid and the surface(s) being coated needs to be <90°, and preferably <45° and most preferably <30°.

Contact angle is one of the common ways to measure the wettability of a surface or material. Wetting refers to the study of how a liquid deposited on a substrate spreads out or the ability of liquids to form boundary surfaces with the substrate. The wetting is determined by measuring the contact angle, which the liquid forms in contact with the substrate. The wetting tendency is larger, the smaller the contact angle or the surface tension is. A wetting liquid is a liquid that forms a contact angle with the solid which is smaller than 90°, whereas, a nonwetting liquid creates a contact angle between 90 and 180° with the solid.

In order for such filling to take place at a reasonable rate, the viscosity of the liquid at ambient temperature is between 0.5 cP and 20 cP, preferably between 1 cP and 10 cP and most preferably between 2 cP and 5 cP.

In the next step, energy is applied to the liquid precursor, causing dissociation of the neutral ligands stabilizing the metal. As these ligands dissociate, the metal ions will begin to coalesce, forming small agglomerates or clusters. As the optional solvent evaporates and more ligands dissociate, these agglomerates continue to grow and concentrate. As these metallic clusters grow, they become nanometer scale particles (nanoparticles). The nanoparticles will concentrate in the recesses of the topography as the solvent and unreacted zerovalent metal-organic liquid evaporate. Then, a conductive film is formed.

A conductive film should have an electrical conductivity at ambient temperature less than or equal (≤) about 1x10⁻⁴ Ωcm. For a 100 Å thick film, this corresponds to a measured sheet resistance less than about 100 Ω/square.

Resistivity of the conductive deposit may be improved by applying energy to the deposited material. Energy is most conveniently applied by external heating using visible or infrared or ultraviolet light or a combination of these radiation sources, through convection using a heated gas stream or by conduction from a resistively or fluid-heated susceptor or from an induction-heated susceptor on which the substrate is placed.

Other sources of energy might also be useful for this process, including electron beams, ion beams, remote hydrogen plasma, direct argon, helium or hydrogen plasma, vacuum and ultrasound.

The conductive film can further undergo a post-deposition annealing treatment.

The post-deposition annealing treatment can be carried out under a reducing atmosphere, including but not limited to hydrogen, ammonia, diborane, silane, at a temperature at or above (≥) 300 °C, for example, from 300 °C to 700 ⁰C; with annealing time of or more than (≥) 5 minutes, for example from 5 to 60 minutes.

The reducing atmospheres can be pure reducing gases or mixtures of the reducing gases with inert gases, such as nitrogen or argon. The pressure of the reducing atmosphere can be at or above (≥) 10 torr, for example, range from 10 torr to 760 torr; and the flow rate of the reducing gas can be at or above (≥) 100 sccm, for example, range from 100-1000 sccm.

In another aspect, the present invention is also a vessel or container employing the metallic precursor comprises at least one neutral (uncharged) metal precursor or at least one neutral (uncharged) metal precursor with a solvent, as defined above.

The method described herein may be used to deposit a conductive film on at least a portion of a substrate. Examples of suitable semiconductor substrates include but are not limited to, silicon, SiO₂, Si₃N₄, OSG, FSG, silicon carbide, hydrogenated silicon oxycarbide, hydrogenated silicon oxynitride, silicon carbo-oxynitride, hydrogenated silicon carbo-oxynitride, antireflective coatings, photoresists, germanium, germanium-containing, boron-containing, Ga/As, a flexible substrate, organic polymers, porous organic and inorganic materials, metals such as copper and aluminum, metal silicide such as titanium silicide, tungsten silicide, molybdenum silicide, nickel silicide, cobalt silicide, and diffusion barrier layers, such as but not limited to cobalt, TiN, Ti(C)N, TaN, Ta(C)N, Ta, W, or WN.

### EXAMPLES

### Example 1

A silicon wafer has a surface layer of carbon-doped silicon oxide into which trenches that are 20 nm wide and 200 nm deep have been etched.

The silicon wafer is situated on a platform in a sealed chamber under inert conditions in a dry oxygen-free nitrogen environment.

Liquid dicobalthexacarbonyltert-butylacetylene (Co₂(CO)₆HC≡CC(CH₃)₃) as the precursor is placed on the silicon wafer.

The pressure of the chamber is reduced first so that any N₂ trapped in the trenches can be removed and the liquid can flow into the trenches by capillary action.

The pressure is then increased by adding nitrogen and then the temperature of the platform is increased gradually.

As the liquid begins to decompose t-butyl acetylene vapors and CO gas will be released and the precursor molecules will begin to oligomerize. The volume of the liquid contracts and the liquid residing on top of the trenches is drawn into the trenches. As condensation continues, solid nanoparticles might form and pack tightly in the trenches.

As the temperature reaches 400 °C, most of the CO and tert-butylacetylene ligands will released into the vapor phase, leaving a conductive Co metal deposit mostly inside the trenches.

Further optional annealing of the deposited material with H₂ gas or by using plasma or electron beams can be employed at this point to increase the conductivity of the metal.

Conventional processing to remove overburden (excess Co on the upper surfaces) such as by chemical mechanical planarization (CMP) can then be performed.

If the trenches are not completely filled, the deposition process may be repeated one or more times until the trenches are completely filled with conductive cobalt metal.

### Example 2

A silicon wafer has a surface layer of carbon-doped silicon oxide into which trenches that are 20 nm wide and 200 nm deep have been etched.

The silicon wafer is situated on a platform in a sealed chamber under inert conditions in a dry oxygen-free nitrogen environment.

Liquid dicobalthexacarbonyltert-butylacetylene (Co₂(CO)₆HC≡CC(CH₃)₃) as the precursor combined with about 10 weight percent dry n-octane is placed on the silicon wafer.

The pressure of the chamber is reduced first so that any N₂ trapped in the trenches can be removed and the liquid can flow into the trenches by capillary action.

The pressure is then increased by adding nitrogen and then the temperature of the platform is increased gradually.

As the liquid begins to decompose t-butyl acetylene vapors and CO gas will be released and the precursor molecules will begin to oligomerize. The volume of the liquid contracts and the liquid residing on top of the trenches is drawn into the trenches. As condensation continues, solid nanoparticles might form and pack tightly in the trenches.

As the temperature reaches 400 °C, most of the CO and tert-butylacetylene ligands will released into the vapor phase, leaving a conductive Co metal deposit mostly inside the trenches.

Further optional annealing of the deposited material with H₂ gas or by using plasma or electron beams can be employed at this point to increase the conductivity of the metal.

Conventional processing to remove overburden (excess Co on the upper surfaces) such as by chemical mechanical planarization (CMP) can then be performed.

If the trenches are not completely filled, the deposition process may be repeated one or more times until the trenches are completely filled with conductive cobalt metal.

### Example 3

### Synthesis of (1-decyne)tetracobalt dodecacarbonyl (Co₄(CO)₁₂(C₈H₁₇C≡CH))

In a nitrogen glovebox, tetracobalt dodecacarbonyl (500 mg, 0.87 mmol) was placed in a 25 cc Schlenk flask. 10 mL Tetrahydrofuran was added into the flask.

Upon stirring, the tetracobalt dodecacarbonyl dissolved to yield a dark solution. 1-Decyne (550 mg, 4.0 mmol) was added to the solution.

The solution was stirred at ambient temperature for 2 days. During this time, the color of the solution changed to dark red.

The volatiles were removed under vacuum to yield a highly viscous black liquid.

### Example 4

### Thermal decomposition of (1-decyne)tetracobalt dodecacarbonyl

In a nitrogen glovebox, a sample of (1-decyne)tetracobalt dodecacarbonyl was placed on a flat pan and transferred to a Thermogravimetric analyzer (TGA).

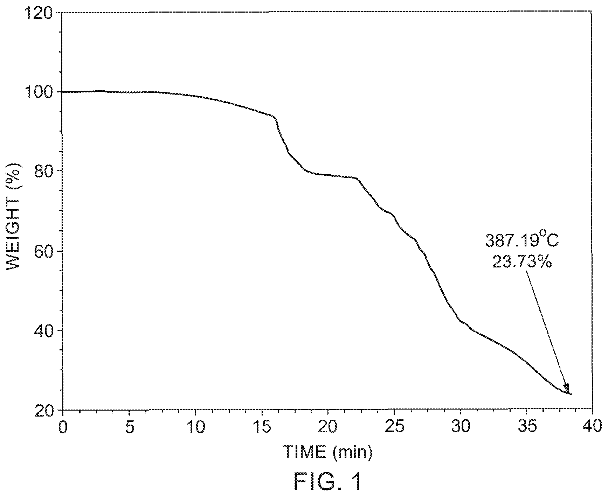

Using the TGA, the temperature of the sample was ramped to 400 °C at 10 °C /minute while monitoring the weight of the sample. A total of 76% of the initial weight was lost, leaving 24% residue (Figure 1). In the compound (1-decyne)tetracobalt dodecacarbonyl, cobalt makes up about 33% of the mass and the ligands make up about 67%. Thus, a majority of the cobalt initially present in the mixture is retained on the surface of the pan.

### Example 5

### Synthesis of Ru₃(CO)₉(PPh₂(CH₂)₃Si(OEt)₃)₃ as a precursor

Ru₃(CO)₁₂ (0.5 g, 0.78 mmol) from Colonial metals inc. and PPh₂(CH₂)₃Si(OEt)₃ (1g, 2.56mmol) from Strem Chemicals are charged into a 250ml flask inside the glovebox. The flask is then moved out of the glovebox and attached to Schlenk line (under N₂).

Under N₂ purge and stirring, anhydrous hexane (100mL) from Sigma-Aldrich is added into the flask with a syringe. The flask is heated under reflux for two hours at 68-70⁰ C. After two hours, the reaction is cooled down to ambient temperature. All solvent is pumped off under vacuum at ambient temperature. The product is washed by cold hexane 3x 10ml. The final product is dried under vacuum. Reddish oil, 0.55g, yield 85% is then obtained.

### Example 6

A mixture of triruthenium dodecacarbonyl with 20% dry n-octane is placed on a silicon wafer having a surface layer of carbon-doped silicon oxide into which trenches that are 20 nm wide and 200 nm deep have been etched. The wafer is sealed in a chamber under inert conditions in a dry oxygen-free nitrogen environment. The pressure of the chamber is reduced so that any N₂ trapped in the trenches can be removed and the liquid can flow into the trenches by capillary action while the solvent begins to evaporate. The pressure is then increased by adding nitrogen and then the temperature of the platform on which the wafer is situated is increased gradually. As the liquid begins to decompose, CO gas will be released and the precursor molecules will begin to oligomerize. The volume of the liquid contracts and the liquid residing on top of the trenches is drawn into the trenches. As condensation continues, solid nanoparticles might form and pack tightly in the trenches. As the temperature reaches 400 °C, most of the CO ligands will released into the vapor phase, leaving a conductive ruthenium metal deposit mostly inside the trenches. Further optional thermal annealing of the deposited material with H₂ or O₂ gas or by using plasma or electron beams can be employed at this point to increase the conductivity of the metal. Conventional processing to remove overburden (excess Ru on the upper surfaces) such as by chemical mechanical planarization (CMP) can then be performed. If the trenches are not completely filled, this process may be repeated one or more times until the trenches are completely filled with conductive ruthenium or a different metal.

### Example 7

(1,6-Heptadiyne) tetracobalt dodecacarbonyl combined with about 10 weight percent dry n-octane is placed on a silicon wafer having a surface layer of carbon-doped silicon oxide into which trenches that are 20 nm wide and 200 nm deep have been etched. The wafer is sealed in a chamber under inert conditions in a dry oxygen-free nitrogen environment. The pressure of the chamber is reduced so that any N₂ trapped in the trenches can be removed and the liquid can flow into the trenches by capillary action while the solvent begins to evaporate. The pressure is then increased by adding nitrogen and then the temperature of the platform on which the wafer is situated is increased gradually. As the liquid begins to decompose, 1,6-heptadiyne vapors and CO gas will be released and the precursor molecules will begin to oligomerize. The volume of the liquid contracts and the liquid residing on top of the trenches is drawn into the trenches. As condensation continues, solid nanoparticles might form and pack tightly in the trenches. As the temperature reaches 400 °C, most of the CO and 1,6-heptadiyne ligands will released into the vapor phase, leaving a conductive Co metal deposit mostly inside the trenches. Further optional annealing of the deposited material with H2 gas or by using plasma or electron beams can be employed at this point to increase the conductivity of the metal. Conventional processing to remove overburden (excess Co on the upper surfaces) such as by chemical mechanical planarization (CMP) can then be performed. If the trenches are not completely filled, this process may be repeated one or more times until the trenches are completely filled with conductive cobalt metal.

### Example 8

### Synthesis of 2,2-Dimethyl-3-octyne (tert-butyl n-butyl acetylene)

In a nitrogen glovebox, a solution of tert-butylacetylene (3,3-dimethyl-1-butyne) was prepared by placing tert-butylacetylene (32.8 g, 0.4 mol) in a 1000 mL round bottom flask with 500 mL of anhydrous THF. To a 500 mL addition funnel was added 150 mL of 2.5 M n-butyllithium in hexanes (0.375 mol). The flask and addition funnel were removed from the glovebox and assembled in the hood. The tert-butylacetylene solution was cooled to 0 °C. The n-butyllithium solution was added dropwise to the tert-butylacetylene solution over 30 minutes with stirring. After the addition was complete, the colorless solution was allowed to warm to ambient temperature over two hours with stirring. To a 500 mL addition funnel was added 1-lodobutane (64.4 g, 0.35 mol) and 100 mL anhydrous THF. This solution was added dropwise to the lithium tert-butylacetylide solution over 30 minutes with stirring. The solution was stirred at ambient temperature for 3 days. GC-MS analysis of a small sample showed complete conversion to the product. The solution was extracted two times with 100 mL of deionized water. The water washes were extracted with 200 mL of hexane and this extract was combined with the THF/hexane solution. The organic solution was dried over magnesium sulfate for 30 minutes. During this time, the colorless solution became light yellow. The combined organic solutions were distilled at reduced pressure (~10 Torr) while holding the reboiler at 20 °C, the condenser at 0 °C, and the collection flask at -78 °C. After the removal of solvent, another collection flask was fitted, and the remaining volatiles distilled while holding the reboiler at 25 °C, the condenser at 0 °C, and the collection flask at -78 °C. The pressure during the second distillation was ~2 torr. When all of the volatiles had been transferred, the collection flask was allowed to warm to ambient temperature. The colorless liquid was analyzed using GC-MS, confirming the presence of highly pure product (>99% purity, 42.2 g, 87% yield).

¹H NMR analysis of 2,2-dimethyl-3-octyne gives the following chemical shifts: 2.03 (t, 2H); 1.33 (m, 4H); 1.19 (s, 9H); 0.80 (t, 3H).

### Example 9

### Synthesis of (2,2-dimethyl-3-octyne) dicobalt hexacarbonyl (Cobalt Carbonyl Tert-butyl N-Butyl Acetylene, CCTNBA)

In a ventilated hood, a solution of 2,2-dimethyl-3-octyne (21.5 g, 0.15 mol) in hexanes (100 mL) was added over 30 minutes to a solution of Co₂(CO)₈ (47.5 g, 0.14 mol) in hexanes (700 mL). Visible CO evolution was observed upon addition of the 2,2-dimethyl-3-octyne solution. The resulting dark brown solution turned dark reddish brown over the course of stirring at ambient temperature for four hours. The hexanes were removed using vacuum distillation while holding the reboiler at 25 °C (condenser temp. - 5 °C; collection flask temp. -78 °C), to yield a dark red liquid with dark solids. A chromatography column (~3 inches in diameter) was packed with 8 inches of neutral activated alumina using pure hexanes as the eluent. The crude material was placed on the column and eluted using hexanes. A brown band quickly moved down the column with the hexanes. Dark purple material was retained in the top 2-3" of the column. The reddish-brown band was collected and evacuated on a Schlenk line (~700 mTorr), yielding 40.0 g of a dark red liquid.

¹H NMR analysis of CCTNBA showed high purity (NMR assay 99.6%). Chemical shifts (d₈-toluene): 2.66 (t, 2H), 1.60 (m, 2H), 1.29 (m, 2H), 1.17 (s, 9H), 0.86 (t, 3H).

### Example 10

### Formation of cobalt-containing films using CCTNBA

In a nitrogen glovebox, ~20 wt. % solutions of CCTNBA were prepared in hexanes and toluene by weighing 250 mg of CCTNBA and 1 g of hexanes/toluene into two 25 mL glass bottles.

Wafer coupons of thermal SiO₂ and silicon of approximate dimensions of 1" x 1" were brought into a nitrogen glovebox. Two coupons of each type were placed in a glass evaporating dish.

The coupons were covered with a thin film of either solution with CCTNBA in hexanes or solution with CCTNBA in toluene by adding the solutions dropwise to the surfaces of the coupons.

The wetting properties of the solutions were slightly different. It took about 5-6 drops of the solution having hexanes s to cover the entire coupon surface. It took 8-9 drops of the solution having toluene to cover the entire coupon surface.

For both sets of solutions, it was possible to cover essentially the entire surface area of the coupons without any of the solutions spilling over the edges of the coupons.

The coupons with the ~20 wt. % solutions of CCTNBA were allowed to stand at room temperature in the glovebox. During this time, the hexanes solutions evaporated entirely. However, the toluene solutions were only partially evaporated.

The glass dish containing the coupons was carefully placed on a heating plate. The heating plate was warmed to 80 °C. After several minutes, it was apparent that the toluene had evaporated and the CCTNBA was still present on the coupon surfaces. After 5 minutes, the dish was removed from the heating plate.

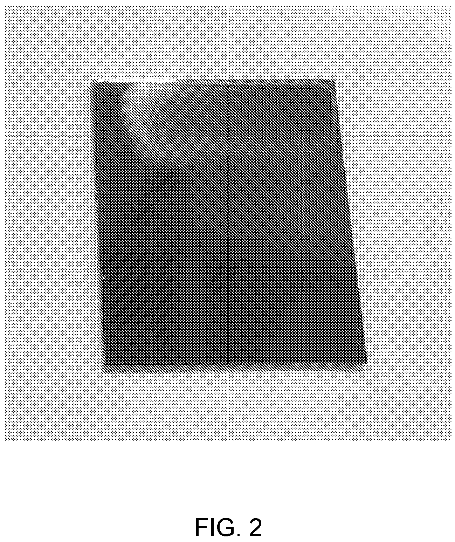

The temperature of the hotplate was increased to 370 °C. When the hotplate surface was stabilized at 370 °C, the dish containing the coupons was placed back on the hotplate. A second evaporating dish of a slightly larger size was placed on top of the dish containing the coupons (acting as a lid). After about 30 seconds, a small amount brown vapor was observed rising from the coupon surfaces. The vapor condensed on the sides of the dish containing the coupons and the part of the larger dish acting as a lid. The coupons were heated for 15 minutes at 370 °C. Within several minutes at 370 °C, the coupon surfaces were mostly shiny silver with some dull grey regions. The hotplate heating was terminated, the glass dish was allowed to cool to ambient temperature. The conductive cobalt-containing films were deposited on the coupons. An example was shown in Figure 2.

The coupons were removed from the dish for analysis.

X-ray fluorescence (XRF) was used to measure the film thickness. A four-point probe was used to measure the film sheet resistance. The sheet resistance was measured after film deposition. The results are shown in Table I.

The coupons were then placed in a chamber for annealing under a hydrogencontaining atmosphere. The conditions for post-deposition annealing treatment were: nitrogen flow 450 sccm, hydrogen flow 50 sccm, temperature 400 °C, chamber pressure 50 torr, anneal time 30 minutes.

The four-point probe was used again to measure the film sheet resistance after the annealing. The results are shown in Table I.

Table I shows the effect of annealing on the resistivity of the deposited cobalt films. The annealing process lowers the resistivity of the cobalt-containing films.

**Table I**

| Wafer surface | Solvent | Film thickness (Angstroms) | Sheet resistance before H₂ anneal (ohms/sq) | Sheet resistance after H₂ anneal (ohms/sq) |
|---|---|---|---|---|
| SiO₂ | Hexanes | 196 | 1300 | 1090 |
| SiO₂ | Toluene | 515 | 2420 | 2160 |
| SiO₂ | Hexanes | 236 | 6790 | 4700 |
| SiO₂ | Toluene | 260 | 1220 | 218 |
| Si | Hexanes | 690 | 154 | 9 |
| Si | Toluene | 618 | 476 | 189 |
| Si | Hexanes | 197 | Not conductive | 197 |
| Si | Toluene | 668 | 494 | 33 |

Films were deposited on both silica and silicon surfaces. Most of the films as deposited contain cobalt and were conductive as measured by a four-point probe measurement apparatus. There appeared to be impurities, such as carbon, in the cobalt films that result in high sheet resistance. Annealing the cobalt films under a reducing atmosphere, such as a mixture of hydrogen and nitrogen, is a method of reducing impurity levels.

The results in Table I demonstrate that the resistivity can be lowered in the films of the current invention. The resulting films may be used to generate a conductive layer or conductive features, such as conductive lines or vias, in semiconductor devices.

While the principles of the invention have been described above in connection with preferred embodiments, it is to be clearly understood that this description is made only by way of example and not as a limitation of the scope of the invention.

## Claims

1. A method to deposit a conductive metallic film onto a substrate comprising:
A. providing the substrate with a surface containing topography comprising patterned recesses;
B. providing liquid metallic precursor comprising a neutral uncharged metal compound selected from the group consisting of
a. R¹CO₂(CO)₆, wherein R¹ is a linear or branched C₂ to C₁₀ alkyne, a linear or branched C₁ to C₁₀ alkoxy alkyne, a linear or branched C₁ to C₁₀ organoamino alkyne, preferably (tert-butylacetylene)dicobalt hexacarbonyl, [Co₂(CO)₆HC≡CC(CH₃)₃];
b. R¹CoFe(CO)₇, wherein R¹ is a linear or branched C₂ to C₁₀ alkyne, a linear or branched C₁ to C₁₀ alkoxy alkyne, a linear or branched C₁ to C₁₀ organoamino alkyne;
c. R²CCo₃(CO)₉, wherein R² is selected from the group consisting of hydrogen, a linear or branched C₁ to C₁₀ alkyl, a linear or branched C₁ to C₁₀ alkoxy, Cl, Br, COOH, COOMe, COOEt;
d. R²CCo₂Mn(CO)₁₀, wherein R² is selected from the group consisting of hydrogen, a linear or branched C₁ to C₁₀ alkyl, a linear or branched C₁ to C₁₀ alkoxy, Cl, Br, COOH, COOMe, COOEt;
e. R³Co₄(CO)₁₂,wherein R³ is selected from a linear or branched C₁ to C₁₀ alkenylidene or wherein the compound is (1-decyne) tetracobalt dodecacarbonyl (Co₄(CO)₁₂(C₈H₁₇C≡CH)), (1,6-heptadiyne) tetracobalt dodecacarbonyl, or (2,2,6-trimethyl-3-heptyne) dicobalt hexacarbonyl; and
f. R⁴Ru₃(CO)₁₁, wherein R⁴ is selected from the group consisting of a disubstituted alkyne (R^{#}CCR^{##}) wherein R^{#} and R^{##} can be selected independently from the group consisting of C₁ to C₁₂ linear, branched, cyclic or aromatic halocarbyl or hydrocarbyl radical, silyl or organosilyl radical, stannyl or organostannyl radical, and combinations thereof; and
the neutral uncharged metal compound is present as a liquid or a solution of a solid soluble at ambient temperature in a solvent selected from the group consisting of saturated linear, branched and cyclic hydrocarbons, o-dichlorobenzene, nitrobenzene; nitriles selected from a group comprising of acetonitrile, propionitrile or benzonitrile; ethers selected from a group comprising of tetrahydrofuran, dimethoxyethane, diglyme, tetrahydropyran, methyltetrahydrofuran, butyltetrahydrofuran, p-dioxane; amines selected from a group comprising of triethylamine, piperidine, pyridine, pyrrolidine, morpholine; amides selected from a group comprising of N,N-dimethylacetamide, N,N-dimethylformamide, N-methylpyrrolidinone, N-cyclohexylpyrrolidinone; aminoethers having formulae R⁴R⁵NR⁶OR⁷NR⁸R⁹, R⁴OR⁶NR⁶R⁹, O(CH₂CH₂)₂NR⁴, R⁴R⁵NR⁶N(CH₂CH₂)₂O, R⁴R⁵NR^{S}OR⁷N(CH₂CH₂)₂O, O(CH₂CH₂)₂NR⁴OR⁶N(CH₂CH₂)₂O; wherein R⁴⁻⁹ are independently selected from the group consisting of a linear or branched C₁ to C₁₀ alkyl; and combinations thereof;
and
the liquid metallic precursor has a viscosity at ambient temperature between 0.5 cP and 20 cP;
C. applying the liquid metallic precursor to the surface to deposit the conductive metallic film onto the substrate by spray coating, roll coating, doctor blade drawdown by squeegee, spin coating, pooling on the surface, inkjet printing, curtain coating, dip-coating, or combinations thereof; and
D. applying vacuum or an energy to the liquid metallic precursor to dissociate the ligands stabilizing the metal; wherein the energy is selected from the group consisting of visible, infrared or ultraviolet light; a heated gas stream; conduction from a resistively or fluid-heated susceptor; an induction-heated susceptor; electron beams; ion beams; remote hydrogen plasma; direct argon; helium or hydrogen plasma; ultrasound; and combinations thereof.

2. The method of Claim 1, wherein the neutral uncharged metal compound is selected from the group consisting of dicobalthexacarbonyl-tert-butylacetylene [Co₂(CO)₆HC≡CC(CH₃)₃], (1-decyne) tetracobalt dodecacarbonyl (Co₄(CO)₁₂(C₈H₁₇C≡CH)), (1,6-heptadiyne) tetracobalt dodecacarbonyl, (2,2,6-trimethyl-3-heptyne) dicobalt hexacarbonyl, (2,2-dimethyl-3-octyne) dicobalt hexacarbonyl (CCTNBA), (2,2-dimethyl-3-heptyne) dicobalt hexacarbonyl, (tert-butylmethylacetylene)dicobalt hexacarbonyl (CCTMA), and combinations thereof.

3. The method of Claim 1 or Claim 2, wherein the solvent is selected from the group consisting of n-hexane, n-pentane, isomeric hexanes, octane, isooctane, decane, dodecane, heptane, cyclohexane, methylcyclohexane, ethylcyclohexane, decalin; aromatic solvent selected from a group comprising of benzene, toluene, single isomer xylene, mixture of xylene isomers, mesitylene, o-dichlorobenzene, nitrobenzene; nitriles selected from a group comprising of acetonitrile, propionitrile or benzonitrile; ethers selected from a group comprising of tetrahydrofuran, dimethoxyethane, diglyme, tetrahydropyran, methyltetrahydrofuran, butyltetrahydrofuran, p-dioxane; amines selected from a group comprising of triethylamine, piperidine, pyridine, pyrrolidine, morpholine; amides selected from a group comprising of N,N-dimethylacetamide, N,N-dimethylformamide, N-methylpyrrolidinone, N-cyclohexylpyrrolidinone; aminoethers having formulae R⁴R⁵NR⁶OR⁷NR⁸R⁹, R⁴OR⁶NR⁸R⁹, O(CH₂CH₂)₂NR⁴, R⁴R⁵NR⁶N(CH₂CH₂)₂O, R⁴R⁵NR⁶OR⁷N(CH₂CH₂)₂O, O(CH₂CH₂)₂NR⁴OR⁶N(CH₂CH₂)₂O; wherein R⁴⁻⁹ are independently selected from the group consisting of a linear or branched C₁ to C₁₀ alkyl; and combinations thereof.

4. The method of Claim 1, wherein the neutral uncharged metal compound is selected from the group consisting of dicobalthexacarbonyltert-butylacetylene [Co₂(CO)₆HC≡CC(CH₃)₃], (1-decyne) tetracobalt dodecacarbonyl (Co₄(CO)₁₂(C₈H₁₇C≡CH)), (1,6-heptadiyne) tetracobalt dodecacarbonyl, (2,2,6-trimethyl-3-heptyne) dicobalt hexacarbonyl, and (2,2-dimethyl-3-octyne) dicobalt hexacarbonyl (CCTNBA);_{;}
and
the solvent is selected from the group consisting of tetrahydrofuran, octane, hexane, toluene.

5. The method of Claim 1, wherein the liquid metallic precursor is applied to the surface with a contact angle between the liquid metallic precursor and the surface at ≤90°.

6. The method of Claim 1, wherein the liquid metallic precursor has a viscosity at ambient temperature between 1 cP and 10 cP; and is applied to the surface with a contact angle between the liquid metallic precursor and the surface at <45°.

7. The method of Claim 1 further comprises applying a post-deposition annealing treatment under a reducing atmosphere using a reducing gas selected from the group consisting of hydrogen, ammonia, diborane, silane, and combinations thereof for an annealing time of 5 minutes or more;
wherein the reducing atmosphere optionally further comprises an inert gas of nitrogen, argon or combinations of nitrogen and argon and the reducing atmosphere is at a temperature equal to or above 300 ⁰C; and the reducing gas is flowing at or above 100 sccm.

8. A system to deposit a conductive metallic film onto a substrate comprising:
a. the substrate with a surface containing topography comprising patterned recesses;
b. liquid metallic precursor as defined in any one of Claims 1 to 4 and
c. a deposition tool selected from the group consisting of spray coating, roll coating, doctor blade drawdown by squeegee, spin coating, pooling on the surface, inkjet printing, curtain coating, dip-coating, and combinations thereof.

9. The system of Claim 8, wherein the liquid metallic precursor has viscosity at ambient temperature between 1 cP and 10 cP.

10. A vessel containing liquid metallic precursor as defined in any one of Claims 1 to 4;
wherein the vessel has a dip-tube extending beneath the surface of the liquid metallic precursor.

11. The vessel of Claim 10, wherein the liquid metallic precursor has viscosity at ambient temperature between 1 cP and 10 cP.

12. A conductive metallic film deposited on a surface containing topography comprising patterned recesses by using liquid metallic precursor comprising
a neutral uncharged metal compound selected from the group consisting of dicobalthexacarbonyltert-butylacetylene [Co₂(CO)₆HC≡CC(CH₃)₃], (1-decyne) tetracobalt dodecacarbonyl (Co₄(CO)₁₂(C₈H₁₇C≡CH)), (1,6-heptadiyne) tetracobalt dodecacarbonyl, (2,2,6-trimethyl-3-heptyne) dicobalt hexacarbonyl, and (2,2-dimethyl-3-octyne) dicobalt hexacarbonyl (CCTNBA);
and
a solvent selected from the group consisting of tetrahydrofuran, octane, hexane, toluene, wherein the film is optionally deposited by spray coating, roll coating, spin coating, inkjet printing, dip-coating, and combinations thereof, and wherein the film optionally has an electrical conductivity less than or equal to 1 x 10⁻⁴ Ωcm at ambient temperature.

## Patentansprüche

1. Verfahren zum Abscheiden eines leitfähigen Metallfilms auf ein Substrat, umfassend:
A. Versehen des Substrats mit einer Oberfläche mit Topographie, die strukturierte Vertiefungen umfasst;
B. Bereitstellen eines flüssigen metallischen Vorläufers, der eine neutrale ungeladene Metallverbindung umfasst, ausgewählt aus der Gruppe bestehend aus
a. R¹Co₂(CO)₆, wobei R¹ ein lineares oder verzweigtes C₂- bis C₁₀-Alkin, ein lineares oder verzweigtes C₁- bis C₁₀-Alkoxyalkin, ein lineares oder verzweigtes C₁- bis C₁₀-Organoaminoalkin, vorzugsweise (tert-Butylacetylen)dicobalthexacarbonyl, [Co₂(CO)₆HC≡CC(CH₃)₃], ist;
b. R¹CoFe(CO)₇, wobei R¹ ein lineares oder verzweigtes C₂- bis C₁₀-Alkin, ein lineares oder verzweigtes C₁- bis C₁₀-Alkoxyalkin, ein lineares oder verzweigtes C₁- bis C₁₀-Organoaminoalkin ist;
c. R²CCo₃(CO)₉, wobei R² ausgewählt ist aus der Gruppe bestehend aus Wasserstoff, einem linearen oder verzweigten C₁- bis C₁₀-Alkyl, einem linearen oder verzweigten C₁- bis C₁₀-Alkoxy, Cl, Br, COOH, COOMe, COOEt;
d. R²CCo₂Mn(CO)₁₀, wobei R² ausgewählt ist aus der Gruppe bestehend aus Wasserstoff, einem linearen oder verzweigten C₁- bis C₁₀-Alkyl, einem linearen oder verzweigten C₁- bis C₁₀-Alkoxy, Cl, Br, COOH, COOMe, COOEt;
e. R³CO₄(CO)₁₂, wobei R³ ausgewählt ist aus einem linearen oder verzweigten C₁- bis C₁₀-Alkenyliden oder wobei die Verbindung (1-Decin)tetracobaltdodecacarbonyl (Co₄(CO)₁₂(C₈H₁₇C≡CH)), (1,6-Heptadiin)tetracobaltdodecacarbonyl oder (2,2,6-Trimethyl-3-heptin)dicobalthexacarbonyl ist; und
f. R⁴Ru₃(CO)₁₁, wobei R⁴ ausgewählt ist aus der Gruppe bestehend aus einem disubstituierten Alkin (R^{#}CCR^{##}), wobei R^{#} und R^{##} unabhängig ausgewählt sein können aus der Gruppe bestehend aus linearem, verzweigtem, cyclischem oder aromatischem C₁- bis C₁₂-Halogencarbyl- oder Hydrocarbylrest, Silyl- oder Organosilylrest, Stannyl- oder Organostannylrest und Kombinationen davon; und
wobei die neutrale ungeladene Metallverbindung vorhanden ist als Flüssigkeit oder Lösung eines bei Raumtemperatur löslichen Feststoffs in einem Lösungsmittel ausgewählt aus der Gruppe bestehend aus gesättigten linearen, verzweigten und cyclischen Kohlenwasserstoffen, o-Dichlorbenzol, Nitrobenzol; Nitrilen ausgewählt aus einer Gruppe bestehend aus Acetonitril, Propionitril oder Benzonitril; Ethern ausgewählt aus einer Gruppe bestehend aus Tetrahydrofuran, Dimethoxyethan, Diglyme, Tetrahydropyran, Methyltetrahydrofuran, Butyltetrahydrofuran, p-Dioxan; Aminen ausgewählt aus einer Gruppe bestehend aus Triethylamin, Piperidin, Pyridin, Pyrrolidin, Morpholin; Amiden ausgewählt aus einer Gruppe bestehend aus N,N-Dimethylacetamid, N,N-Dimethylformamid, N-Methylpyrrolidinon, N-Cyclohexylpyrrolidinon; Aminoethern mit den Formeln R⁴R⁵NR⁶OR⁷NR⁸R⁹, R⁴OR⁶NR⁸R⁹, O(CH₂CH₂)₂NR⁴, R⁴R⁵NR⁶N(CH₂CH₂)₂O, R⁴R⁵NR⁶OR⁷N(CH₂CH₂)₂O, O(CH₂CH₂)₂NR⁴OR⁶N(CH₂CH₂)₂O; wobei R⁴⁻⁹ unabhängig ausgewählt sind aus der Gruppe bestehend aus einem linearen oder verzweigten C₁- bis C₁₀-Alkyl; und Kombinationen davon;
und
der flüssige metallische Vorläufer eine Viskosität bei Umgebungstemperatur zwischen 0,5 cP und 20 cP aufweist;
C. Aufbringen des flüssigen metallischen Vorläufers auf die Oberfläche, um den leitfähigen metallischen Film auf das Substrat abzuscheiden, durch Sprühbeschichten, Walzenbeschichten, Rakelabziehen durch Rakeln, Schleuderbeschichten, Vereinigen auf der Oberfläche, Tintenstrahldruck, Vorhangbeschichten, Tauchbeschichten oder Kombinationen davon; und
D. Anlegen von Vakuum oder einer Energie an den flüssigen metallischen Vorläufer, um die Liganden, die das Metall stabilisieren, zu dissoziieren; wobei die Energie ausgewählt ist aus der Gruppe bestehend aus sichtbarem, infrarotem oder ultraviolettem Licht; einem erhitzten Gasstrom; Leitung von einem resistiven oder fluiderwärmten Suszeptor; einem induktionserwärmten Suszeptor; Elektronenstrahlen; Ionenstrahlen; entferntem Wasserstoffplasma; direktem Argon; Helium- oder Wasserstoffplasma; Ultraschall; und Kombinationen davon.

2. Verfahren nach Anspruch 1, wobei die neutrale ungeladene Metallverbindung ausgewählt ist aus der Gruppe bestehend aus Dicobalthexacarbonyl-tert-butylacetylen [Co₂(CO)₆HC=CC(CH₃)₃], (1-Decin)tetracobaltdodecacarbonyl (Co₄(CO)₁₂(C₈H₁₇C≡CH)), (1,6-Heptadiin)tetracobaltdodecacarbonyl, (2,2,6-Trimethyl-3-heptin)dicobalthexacarbonyl, (2,2-Dimethyl-3-octin)dicobalthexacarbonyl (CCTNBA), (2,2-Dimethyl-3-heptin)dicobalthexacarbonyl, (tert-Butylmethylacetylen)dicobalthexacarbonyl (CCTMA) und Kombinationen davon.

3. Verfahren nach Anspruch 1 oder Anspruch 2, wobei das Lösungsmittel ausgewählt ist aus der Gruppe bestehend aus n-Hexan, n-Pentan, isomeren Hexanen, Octan, Isooctan, Decan, Dodecan, Heptan, Cyclohexan, Methylcyclohexan, Ethylcyclohexan, Decalin; aromatischem Lösungsmittel ausgewählt aus einer Gruppe umfassend Benzol, Toluol, einzelisomeres Xylol, Gemisch von Xylolisomeren, Mesitylen, o-Dichlorbenzol, Nitrobenzol; Nitrilen ausgewählt aus einer Gruppe umfassend Acetonitril, Propionitril oder Benzonitril; Ether ausgewählt aus einer Gruppe umfassend Tetrahydrofuran, Dimethoxyethan, Diglyme, Tetrahydropyran, Methyltetrahydrofuran, Butyltetrahydrofuran, p-Dioxan; Aminen ausgewählt aus einer Gruppe umfassend Triethylamin, Piperidin, Pyridin, Pyrrolidin, Morpholin; Amiden ausgewählt aus einer Gruppe umfassend N,N-Dimethylacetamid, N,N-Dimethylformamid, N-Methylpyrrolidinon, N-Cyclohexylpyrrolidinon; Aminoethern mit den Formeln R⁴R⁵NR⁶OR⁷NR⁸R⁹, R⁴OR⁶NR⁸R⁹, O(CH₂CH₂)₂NR⁴, R⁴R⁵NR⁶N(CH₂CH₂)₂O, R⁴R⁵NR⁶OR⁷N(CH₂CH₂)₂O, O(CH₂CH₂)₂NR⁴OR⁶N(CH₂CH₂)₂O; wobei R⁴⁻⁹ unabhängig ausgewählt sind aus der Gruppe bestehend aus einem linearen oder verzweigten C₁- bis C₁₀-Alkyl; und Kombinationen davon.

4. Verfahren nach Anspruch 1, wobei die neutrale ungeladene Metallverbindung ausgewählt ist aus der Gruppe bestehend aus Dicobalthexacarbonyl-tert-butylacetylen [Co₂(CO)₆HC≡CC(CH₃)₃], (1-Decin)tetracobaltdodecacarbonyl (Co₄(CO)₁₂(C₈H₁₇C≡CH)), (1,6-Heptadiin)tetracobaltdodecacarbonyl, (2,2,6-Trimethyl-3-heptin)dicobalthexacarbonyl und (2,2-Dimethyl-3-octin)dicobalthexacarbonyl (CCTNBA);
und
das Lösungsmittel ausgewählt ist aus der Gruppe bestehend aus Tetrahydrofuran, Octan, Hexan, Toluol.

5. Verfahren nach Anspruch 1, wobei der flüssige metallische Vorläufer mit einem Kontaktwinkel zwischen dem flüssigen metallischen Vorläufer und der Oberfläche von ≤90° auf die Oberfläche aufgebracht wird.

6. Verfahren nach Anspruch 1, wobei der flüssige metallische Vorläufer eine Viskosität bei Umgebungstemperatur zwischen 1 cP und 10 cP aufweist; und mit einem Kontaktwinkel zwischen dem flüssigen metallischen Vorläufer und der Oberfläche von <45° auf die Oberfläche aufgebracht wird.

7. Verfahren nach Anspruch 1, ferner umfassend Anwenden einer Nachabscheidungs-Temperbehandlung unter einer reduzierenden Atmosphäre unter Verwendung eines reduzierenden Gases ausgewählt aus der Gruppe bestehend aus Wasserstoff, Ammoniak, Diboran, Silan und Kombinationen davon, für eine Temperzeit von 5 Minuten oder mehr;
wobei die reduzierende Atmosphäre gegebenenfalls ferner ein Inertgas aus Stickstoff, Argon oder Kombinationen von Stickstoff und Argon umfasst und die reduzierende Atmosphäre eine Temperatur von gleich oder über 300 °C aufweist; und das reduzierende Gas mit oder über 100 sccm strömt.

8. System zum Abscheiden eines leitfähigen Metallfilms auf ein Substrat, umfassend:
a. das Substrat mit einer Topographie enthaltenden Oberfläche, die strukturierte Vertiefungen umfasst;
b. flüssigen metallischen Vorläufer nach einem der Ansprüche 1 bis 4 und
c. ein Abscheidewerkzeug ausgewählt aus der Gruppe bestehend aus Sprühbeschichtung, Walzenbeschichtung, Rakelabzug durch Rakeln, Schleuderbeschichtung, Vereinigen auf der Oberfläche, Tintenstrahldruck, Vorhangbeschichtung, Tauchbeschichtung und Kombinationen davon.

9. System nach Anspruch 8, wobei der flüssige metallische Vorläufer eine Viskosität bei Umgebungstemperatur zwischen 1 cP und 10 cP aufweist.

10. Gefäß, das einen flüssigen metallischen Vorläufer nach einem der Ansprüche 1 bis 4 enthält;
wobei das Gefäß ein Tauchrohr aufweist, das sich unter die Oberfläche des flüssigen metallischen Vorläufers erstreckt.

11. Gefäß nach Anspruch 10, wobei der flüssige metallische Vorläufer eine Viskosität bei Umgebungstemperatur zwischen 1 cP und 10 cP aufweist.

12. Leitfähiger Metallfilm, abgeschieden auf eine Oberfläche, die Topographie enthält, die strukturierte Vertiefungen umfasst, unter Verwendung eines flüssigen metallischen Vorläufers umfassend
eine neutrale ungeladene Metallverbindung ausgewählt ist aus der Gruppe bestehend aus Dicobalthexacarbonyl-tert-butylacetylen [Co₂(CO)₆HC≡CC(CH₃)₃], (1-Decin)tetracobaltdodecacarbonyl (Co₄(CO)₁₂(C₈H₁₇C≡CH)), (1,6-Heptadiin)tetracobaltdodecacarbonyl, (2,2,6-Trimethyl-3-heptin)dicobalthexacarbonyl und (2,2-Dimethyl-3-octin)dicobalthexacarbonyl (CCTNBA);
und
ein Lösungsmittel ausgewählt aus der Gruppe bestehend aus Tetrahydrofuran, Octan, Hexan, Toluol, wobei der Film gegebenenfalls durch Sprühbeschichten, Walzenbeschichten, Schleuderbeschichten, Tintenstrahldrucken, Tauchbeschichten und Kombinationen davon abgeschieden wird und wobei der Film gegebenenfalls eine elektrische Leitfähigkeit von weniger als oder gleich 1 x 10⁻⁴ Ωcm bei Umgebungstemperatur aufweist.

## Revendications

1. Procédé de dépôt d'un film métallique conducteur sur un substrat comprenant :
A. fourniture au substrat d'une surface contenant une topographie comprenant des évidements à structures ;
B. fourniture d'un précurseur métallique liquide comprenant un composé métallique neutre non chargé choisi dans le groupe constitué par
a. R¹Co₂(CO)₆, dans lequel R¹ est un alcyne en C₂ à C₁₀ linéaire ou ramifié, un alcoxyalcyne en C₁ à C₁₀ linéaire ou ramifié, un organoaminoalcyne en C₁ à C₁₀ linéaire ou ramifié, de préférence le dicobalthexacarbonyl(tert-butylacétylène), [Co₂(CO)₆HC≡CC(CH₃)₃] ;
b. R¹CoFe(CO)₇, dans lequel R¹ est un alcyne en C₂ à C₁₀ linéaire ou ramifié, un alcoxyalcyne en C₁ à C₁₀ linéaire ou ramifié, un organoaminoalcyne en C₁ à C₁₀ linéaire ou ramifié ;
c. R²CCo₃(CO)₉, dans lequel R² est choisi dans le groupe constitué par hydrogène ou un alkyle en C₁ à C₁₀ linéaire ou ramifié, un alcoxy en C₁ à C₁₀ linéaire ou ramifié, Cl, Br, COOH, COOMe, COOEt ;
d. R²CCo₂Mn(CO)₁₀, dans lequel R² est choisi dans le groupe constitué par hydrogène, un alkyle en C₁ à C₁₀ linéaire ou ramifié, un alcoxy en C₁ à C₁₀ linéaire ou ramifié, Cl, Br, COOH, COOMe, COOEt ;
e. R³CO₄(CO)₁₂, dans lequel R³ est choisi parmi un alcénylidène en C₁ à C₁₀ linéaire ou ramifié ou dans lequel le composé est le (1-décyne)tétracobaltdodécacarbonyle (CO₄(CO)₁₂(C₈H₁₇C≡CH)), (1,6-heptadiyne)tétracobaltdodécacarbonyle ou (2,2,6-triméthyl-3-heptyne)dicobalthexacarbonyle ; et
f. R⁴Ru₃(CO)₁₁, dans lequel R⁴ est choisi dans le groupe constitué par un alcyne disubstitué (R^{#}CCR^{##}) , dans lequel R^{#} et R^{##} peuvent être choisis indépendamment dans le groupe constitué par un radical halogénocarbyle ou hydrocarbyle cyclique, aromatique, linéaire ou ramifié en C₁ à C₁₂, un radical silyle ou organosilyle, un radical stannyle ou organostannyle, et des combinaisons correspondantes ; et
le composé métallique neutre non chargé est présent sous forme de liquide ou de solution d'un solide soluble à température ambiante dans un solvant choisi dans le groupe constitué par les hydrocarbures linéaires, ramifiés et cycliques saturés, l'o-dichlorobenzène, le nitrobenzène ; les nitriles choisis dans un groupe comprenant l'acétonitrile, le propionitrile ou le benzonitrile ; les éthers choisis dans un groupe comprenant le tétrahydrofurane, le diméthoxyéthane, le diglyme, le tétrahydropyrane, le méthyltétrahydrofurane, le butyltétrahydrofurane, le p-dioxane ; les amines choisies dans un groupe comprenant la triéthylamine, la pipéridine, la pyridine, la pyrrolidine, la morpholine ; les amides choisis dans un groupe comprenant le N,N-diméthylacétamide, le N,N-diméthylformamide, la N-méthylpyrrolidinone, la N-cyclohexylpyrrolidinone ; les aminoéthers ayant les formules R⁴R⁵NR⁶OR⁷NR⁸R⁹, R⁴OR⁶NR⁸R⁹, O(CH₂CH₂)₂NR⁴, R⁴R⁵NR⁶N(CH₂CH₂)₂O, R⁴R⁵NR⁶OR⁷N(CH₂CH₂)₂O, O(CH₂CH₂)₂NR⁴OR⁶N(CH₂CH₂)₂O ; dans lesquelles R⁴⁻⁹ sont indépendamment choisis dans le groupe constitué par un alkyle en C₁ à C₁₀ linéaire ou ramifié ; et des combinaisons correspondantes ;
et
le précurseur métallique liquide présente une viscosité à température ambiante comprise entre 0,5 cP et 20 cP ;
C. application du précurseur métallique liquide sur la surface pour déposer le film métallique conducteur sur le substrat par revêtement par pulvérisation, revêtement au rouleau, par une raclette, revêtement par centrifugation, accumulation sur la surface, impression à jet d'encre, revêtement au rideau, revêtement par trempage ou des combinaisons correspondantes ; et
D. application de vide ou d'une énergie au précurseur métallique liquide pour dissocier les ligands stabilisant le métal ; l'énergie étant choisie dans le groupe constitué par une lumière visible, infrarouge ou ultraviolette ; un flux de gaz chauffé ; la conduction à partir d'un suscepteur chauffé par résistance ou par un fluide ; un suscepteur chauffé par induction ; des faisceaux d'électrons ; des faisceaux d'ions ; un plasma d'hydrogène distant ; de l'argon direct ; un plasma d'hélium ou d'hydrogène ; des ultrasons ; et des combinaisons correspondantes.

2. Procédé selon la revendication 1, dans lequel le composé métallique neutre non chargé est choisi dans le groupe constitué par dicobalthexacarbonyl-tert-butylacétylène [Co₂(CO)₆HC≡CC(CH₃)₃], (1-décyne)tétracobaltdodécacarbonyle (Co₄(CO)₁₂(C₈H₁₇C≡CH)), (1,6-heptadiyne)tétracobaltdodécacarbonyle, (2,2,6-triméthyl-3-heptyne)dicobalthexacarbonyle, (2,2-diméthyl-3-octyne)dicobalthexacarbonyle (CCTNBA), (2,2-diméthyl-3-heptyne)dicobalthexacarbonyle, (tert-butylméthylacétylène)dicobalthexacarbonyle (CCTMA), et des combinaisons correspondantes.

3. Procédé selon la revendication 1 ou la revendication 2, dans lequel le solvant est choisi dans le groupe constitué par le n-hexane, le n-pentane, les hexanes isomériques, l'octane, l'isooctane, le décane, le dodécane, l'heptane, le cyclohexane, le méthylcyclohexane, l'éthyl-cyclohexane, la décaline ; un solvant aromatique choisi dans un groupe comprenant le benzène, le toluène, un seul isomère du xylène, un mélange d'isomères de xylènes, le mésitylène, l'o-dichlorobenzène, le nitrobenzène ; les nitriles choisis dans un groupe comprenant l'acétonitrile, le propionitrile ou le benzonitrile ; les éthers choisis dans un groupe comprenant le tétrahydrofurane, le diméthoxyéthane, le diglyme, le tétrahydropyrane, le méthyltétrahydrofurane, le butyltétrahydrofurane, le p-dioxane ; les amines choisies dans un groupe comprenant la triéthylamine, la pipéridine, la pyridine, la pyrrolidine, la morpholine ; les amides choisis dans un groupe comprenant le N,N-diméthylacétamide, le N,N-diméthylformamide, la N-méthylpyrrolidinone, la N-cyclohexylpyrrolidinone ; les aminoéthers de formules R⁴R⁵NR⁶OR⁷NR⁸R⁹, R⁴OR⁶NR⁸R⁹, O(CH₂CH₂)₂NR⁴, R⁴R⁵NR⁶N(CH₂CH₂)₂O, R⁴R⁵NR⁶OR⁷N(CH₂CH₂)₂O, O(CH₂CH₂)₂NR⁴OR⁶N(CH₂CH₂)₂O ; dans lesquelles R⁴⁻⁹ sont choisis indépendamment dans le groupe constitué par un alkyle en C₁ à C₁₀ linéaire ou ramifié ; et des combinaisons correspondantes.

4. Procédé selon la revendication 1, dans lequel le composé métallique neutre non chargé est choisi dans le groupe constitué par dicobalthexacarbonyltert-butylacétylène [Co₂(CO)₆HC≡CC(CH₃)₃], (1-décyne)tétracobaltdodécacarbonyle (Co₄(CO)₁₂(C₈H₁₇C≡CH)), (1,6-heptadiyne)tétracobaltdodécacarbonyle, (2,2,6-triméthyl-3-heptyne)dicobalthexacarbonyle, et (2,2-diméthyl-3-octyne)dicobalthexacarbonyle (CCTNBA) ;
et
le solvant est choisi dans le groupe constitué par le tétrahydrofurane, l'octane, l'hexane, le toluène.

5. Procédé selon la revendication 1, dans lequel le précurseur métallique liquide est appliqué sur la surface avec un angle de contact entre le précurseur métallique liquide et la surface ≤ 90°.

6. Procédé selon la revendication 1, dans lequel le précurseur métallique liquide a une viscosité à température ambiante comprise entre 1 cP et 10 cP ; et est appliqué sur la surface avec un angle de contact entre le précurseur métallique liquide et la surface < 45°.

7. Procédé selon la revendication 1, comprenant en outre l'application d'un traitement de recuit post-dépôt sous une atmosphère réductrice en utilisant un gaz réducteur choisi dans le groupe constitué par l'hydrogène, l'ammoniac, le diborane, le silane, et des combinaisons correspondantes pendant un temps de recuit de 5 minutes ou plus ;
dans lequel l'atmosphère réductrice comprend facultativement en outre un gaz inerte d'azote, d'argon ou de combinaisons d'azote et d'argon et l'atmosphère réductrice est à une température égale ou supérieure à 300 °C ; et le gaz réducteur s'écoule à une vitesse supérieure ou égale à 100 sccm.

8. Système pour déposer un film métallique conducteur sur un substrat comprenant :
a. le substrat avec une surface contenant une topographie comprenant des évidements à structures ;
b. un précurseur métallique liquide tel que défini selon l'une quelconque des revendications 1 à 4 et
c. un outil de dépôt choisi parmi le groupe constitué par un revêtement par pulvérisation, revêtement au rouleau, par une raclette, revêtement par centrifugation, accumulation sur la surface, impression à jet d'encre, revêtement au rideau, revêtement par trempage ou des combinaisons correspondantes.

9. Système selon la revendication 8, dans lequel le précurseur métallique liquide a une viscosité à température ambiante comprise entre 1 cP et 10 cP.

10. Récipient contenant un précurseur métallique liquide tel que défini dans l'une quelconque des revendications 1 à 4 ;
dans lequel le récipient possède un tube plongeur s'étendant sous la surface du précurseur métallique liquide.

11. Récipient selon la revendication 10, dans lequel le précurseur métallique liquide a une viscosité à température ambiante comprise entre 1 cP et 10 cP.

12. Film métallique conducteur déposé sur une surface contenant une topographie comprenant des évidements à structures en utilisant un précurseur métallique liquide comprenant
un composé métallique non chargé neutre choisi dans le groupe constitué par le dicobalthexacarbonyltert-butylacétylène [Co₂(CO)₆HC≡CC(CH₃)₃], (1-décyne)tétracobaltdodécacarbonyle (Co₄(CO)₁₂(C₈H₁₇C≡CH)), (1,6-heptadiyne)tétracobaltdodécacarbonyle, (2,2,6-triméthyl-3-heptyne)dicobalthexacarbonyle, et (2,2-diméthyl-3-octyne)dicobalthexacarbonyle (CCTNBA) ;
et
un solvant choisi dans le groupe constitué par le tétrahydrofurane, l'octane, l'hexane, le toluène, le film étant éventuellement déposé par revêtement par pulvérisation, revêtement au rouleau, revêtement par centrifugation, impression à jet d'encre, revêtement par trempage, et des combinaisons correspondantes, le film ayant éventuellement une conductivité électrique inférieure ou égale à 1 x 10⁻⁴ Ωcm à température ambiante.
